# EUROPÄISCHE PATENTSCHRIFT

(11) **EP 1 739 461 B1**
(45) Veröffentlichungstag und Bekanntmachung des Hinweises auf die Patenterteilung: **20.10.2010**
(21) Anmeldenummer: 05014074.8
(22) Anmeldetag: 29.06.2005
(51) Int. Cl.: G02B 3/14, A61B 3/09

(54) **Vorrichtung und Verfahren zum Untersuchen der Akkommodationsfähigkeit eines Auges**
Device and method for examining the accommodation of an eye
Appareil et méthode pour l'examen de l'accommodation oculaire

(43) Veröffentlichungstag der Anmeldung: 03.01.2007
(73) Patentinhaber: WaveLight GmbH, 91058 Erlangen (DE)
(72) Erfinder: Götzinger, Volker, 90562 Heroldsberg (DE); Schnalke, Andreas, 90491 Nürnberg (DE); Michling, Arthur, 90766 Fürth (DE); Heiberger, Kurt, 90455 Nürnberg (DE)
(74) Vertreter: von Hellfeld, Axel

(56) Entgegenhaltungen:
- WO-A-2004/049927
- WO-A-2005/088610
- DE-A1- 19 623 270
- US-A1- 2002 196 558
- US-A1- 2004 227 063
- US-A1- 2005 002 113
- US-B1- 6 369 954
- PATENT ABSTRACTS OF JAPAN Bd. 013, Nr. 391 (P-926), 30. August 1989 (1989-08-30) -& JP 01 140118 A (MITSUBISHI HEAVY IND LTD), 1. Juni 1989 (1989-06-01)

## Beschreibung

Die Erfindung betrifft eine Vorrichtung zum Untersuchen der Akkommodationsfähigkeit eines Anges.

Der Stand der Technik (Produkte der Firmen Varioptic und Philips; US 6,369,954 und EP 1 019 758) kennt steuerbare Flüssigkeitslinsen, mit denen die Brechkraft im Bereich von etwa -15 dpt bis 30 dpt innerhalb von Zeitintervallen von wenigen Millisekunden mit Spannungen von bis zu 100 V steuerbar sind.

Mit diesen bekannten Flüssigkeitslinsen lässt sich in der Regel jedoch nur eine sphärische Linse mit wenig Linsenfehlern einstellen.

Die WO 2004/049927 A1 zeigt ein Flüssigkeitslinsensystem mit
- einem Flüssigkeitstropfen, dessen Form durch elektrische Felder beeinflussbar ist;
- einer Mehrzahl von Elektroden, die um den Flüssigkeitstropfen herum angeordnet sind;
- und einer elektrischen Spannungsversorgung, um wahlweise zur Erzeugung unterschiedlicher elektrischer Felder verschiedene Spannungen an die Elektroden anzulegen.

Die DE-A-3835 476 A1 zeigt eine Vorrichtung zum Augentraining mit einem oszillierenden Bild, das in ein Auge abgebildet wird.

Der Erfindung liegt die Aufgabe zu grunde, eine Vorrichtung bereitzustellen, mit der die Akkommodationsfähigkeit eines Auges einfach zu untersuchen ist.

Hierzu lehrt die Erfindung eine Vorrichtung gemäss Anspruch 1.

Bevorzugte Ausgestaltungen der Vorrichtung sind in den abhängigen Ansprüchen beschrieben.

Nachfolgend werden Ausführungsbeispiele anhand der Zeichnung näher beschrieben. Es zeigt:
- Figur 1: schematisch einen Schnitt durch ein Flüssigkeitslinsensystem;
- Figur 2: schematisch eine Draufsicht auf die Elektrodenanordnung eines Flüssigkeitslinsensystems gemäß Figur 1;
- Figur 3: ein Akkommodationsuntersuchungsgerät; und
- Figur 4: ein Dioptriefernrohr.

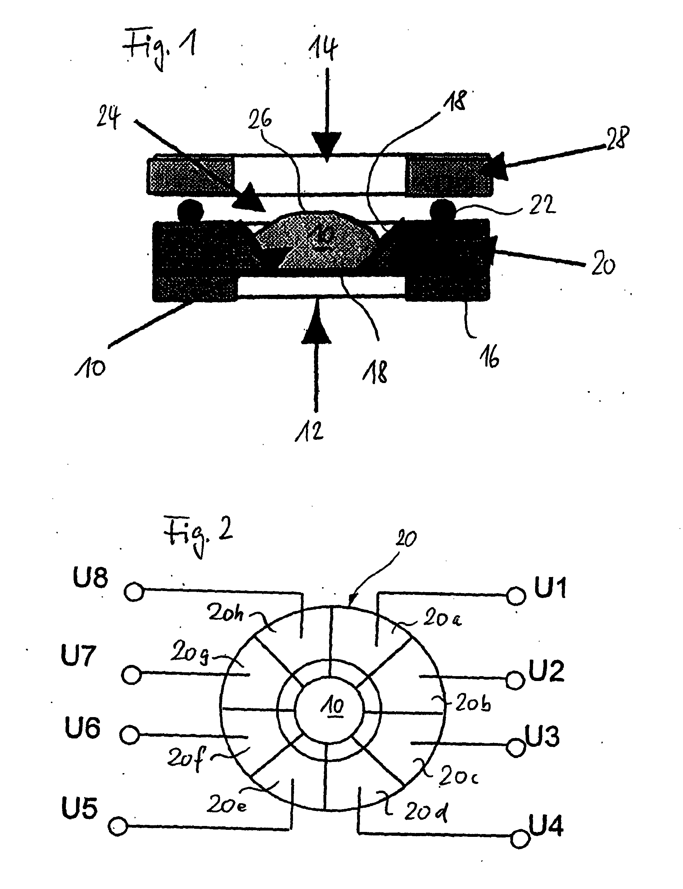

Gemäß Figur 1 ist ein Flüssigkeitstropfen 10, beim dargestellten Ausführungsbeispiel ein Öltropfen, zwischen zwei strahlungsdurchlässigen Fenstern 12, 14 angeordnet. Abzubildende Strahlung (Licht) wird durch den Flüssigkeitstropfen 10 in einstellbarer Weise gebrochen.

Der Flüssigkeitstropfen 10 ruht auf einer transparenten Zwischenlage 18, die wiederum von einem Stützring 16 abgestützt ist.

Um den Flüssigkeitstropfen 10 herum ist ein Elektrodenring 20 angeordnet, der in Figur 2 in Draufsicht dargestellt ist. Wie Figur 2 zeigt, besteht der Elektrodenring 20 beim dargestellten Ausführungsbeispiel aus acht Segmenten (20a, 20b, 20c, 20d, 20e, 20f, 20g, 20h). Die genannten Segmente bilden einen geschlossenen Kreis. Figur 2 zeigt auch die jeweils an die einzelnen Segmenten selektiv anlegbaren elektrischen Spannungen U1, U2, U3, U4, U5, U6, U7 und U8. Durch Anlegung unterschiedlicher Spannungen an die einzelnen Segmente lässt sich die Oberfläche 26 des Flüssigkeitstropfens 10 ändern, sodass die Brecheigenschaften des Flüssigkeitstropfens 10 wahlweise einstellbar sind. Ein Ring 28 bildet eine, z.B. an "Masse" liegende, Gegenelektrode für die einzelnen Elektroden 20a, ..., 20h. Um zum Beispiel eine Zylinderlinse nachzubilden, müssen die Spannungen U2, U3, U6, U7 auf ein erstes Spannungspotential gelegt werden und die Spannungen U1, U4, U5 und U8 der anderen Segmente auf ein anderes Spannungspotential. Auf diese Weise lässt sich mit einem Flüssigkeitslinsensystem gemäß den Figuren 1 und 2 ein Linsenfehler elektrische einstellen, zum Beispiel eine Sphäre, ein Zylinder, Koma, oder auch weitere Fehler höherer Ordnung. Je mehr Segmente analog Figur 2 um den Flüssigkeitstropfen 10 herum angeordnet sind, umso höher ist die örtliche Auflösung der einzustellenden Linsenfehler. Die Anordnung der einzelnen Elektrodensegmente (20a, ..., 20h) muss nicht notwendig rotationssymmetrisch sein. Das Ausführungsbeispiel gemäß Figur 2 zeigt zwar eine acht-zählige Rotationssymmetrie der Elektroden, derart, dass jeweils zwei Elektroden diametral in Bezug auf den Flüssigkeitstropfen 10 liegen. In Abwandlung dieses Ausführungsbeispiels kann, insbesondere zur Erzeugung von asphärischen Verformungen des Flüssigkeitstropfens 10, vorgesehen sein, die Elektroden, abweichend von der Darstellung gemäß Figur 2 so anzuordnen, dass zur Korrektur von typischen Abbildungsfehlern des Auges in Bezug auf die optische Achse asymetrische Verformungen und auch lokal in Abhängigkeit vom Sehfehler des Patienten gezielt Verformungen erreicht werden. Es wird also eine gezielte Verformung der Grenzfläche 26 der Flüssigkeitslinse 10 erzielt.

Nachfolgend werden unterschiedliche Anwendungsbeispiele für Flüssigkeitslinsensysteme der vorstehend beschriebenen Art dargestellt.

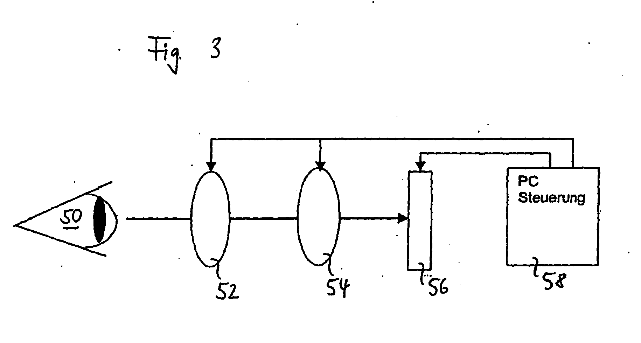

Figur 3 zeigt eine Anwendung von Flüssigkeitslinsensystemen gemäß den Figuren 1 und 2 in einem Akkommodationsuntersuchungsgerät.

Das Gerät gemäß Figur 3 ist relativ einfach aufgebaut und ermöglicht die Messung der Akkommodationsfähigkeit eines menschlichen Auges mit hoher Genauigkeit.

Ein Gegenstand wird auf der Netzhaut des zu untersuchenden Auges 50 als Bild über zwei Flüssigkeitslinsensysteme 52, 54 abgebildet. Der Bildschirm 56 fungiert gleichzeitig als Gegenstand und als Target. Als Bildschirm 56 findet bevorzugt ein TFT-Bildschirm Verwendung, alternativ auch zum Beispiel ein CRT-Bildschirm. Bei der Messung brauchen keine mechanisch beweglichen Teile eingesetzt zu werden. Ein Rechner 58 steuert die beiden Flüssigkeitslinsensysteme 52, 54 und den Bildschirm 56. Diese Anordnung ermöglicht besonders vorteilhaft, dass mit dem Bildschirm 56 die Helligkeit und die Bildgröße so gesteuert werden, dass das abgebildete Bild unabhängig von der Fehlsichtigkeit des untersuchten Auges gleiche Helligkeit und Größe behällt. Damit ist eine genauere Messung der Akkommodationsfähigkeit möglich. Zur Messung der Akkommodationsfähigkeit kann zum Beispiel durch Anlegung geeigneter Spannungen U1, ...., U8 mittels des Rechners 58 mit dem ersten Flüssigkeitslinsensystem 52 eine Sphäre im Millisekundenbereich verändert werden. Synchron kann mit dem Flüssigkeitslinsensystem 54 der Zylinder kompensiert werden. Durch Änderung der Frequenz der erzeugten Bildänderung und Untersuchung des auf der Netzhaut abgebildeten Bildes mittels des Bildschirmes 56 kann dann die Akkommodationsfähigkeit des untersuchten Auges 50 ermittelt werden.

Durch Hinzufügung von weiteren Flüssigkeitslinsensystemen oder auch normalen Linsen zur Anordnung gemäß Figur 3 kann der Messbereich und auch die Messgenauigkeit erhöht werden.

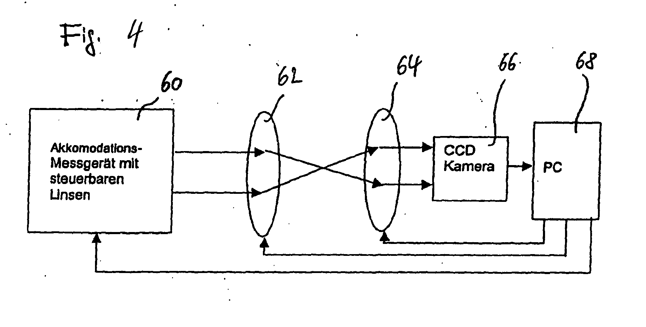

Figur 4 zeigt eine Anordnung, die als Dioptriefernrohr verwendbar ist, also ein Gerät zur Bestimmung des Dioptriewertes eines zu untersuchenden Auges. In Figur 4 ist der Block 60 als Akkommodationsuntersuchungsgerät mit steuerbaren Linsen ausgewiesen, der Block 60 kann also ein System darstellen, wie es vorstehend anhand der Figur 3 beschrieben ist. Mit der Anordnung gemäß Figur 4 kann z.B. ein Akkommodationsuntersuchungsgerät 60 überprüft und kalibriert werden.

Nach Figur 4 sind im Strahlengang nach dem Akkommodationsuntersuchungsgerät zwei Flüssigkeitslinsensysteme 62, 64 angeordnet. Die damit erzeugte Strahlung wird in eine CCD-Kamera 66 abgebildet. Die Bildsignale der Kamera 66 werden in einem Rechner 68 verarbeitet. Der Rechner 68 steuert in der durch Pfeile angedeuteten Weise das Akkommodationsuntersuchungsgerät, das Festkörperlinsensystem 62 und das Festkörperlinsensystem 64, bei Letzteren also die oben erläuterten einstellbaren Spannungen U1, ..., U8.

Mit den genannten Flüssigkeitslinsensystemen lassen sich sowohl die Brechkraft einstellen als auch der Zylinder kompensieren. Eine Software im Rechner 68 wertet das digital gewonnene Bild aus.

Die Anordnung gemäß Figur 4 ist klein und kompakt. Sie ist ausschließlich elektrisch steuerbar und ermöglicht eine einfache Kalibrierung, womit auch eine Zeitersparnis bei der Kalibrierung verbunden ist. Auf eine aufwendige Mechanik und Optik kann also verzichtet werden. Auch ermöglicht der Aufbau gemäß Figur 4 eine einfache Dokumentierung des Kalibriervorganges mittels des Rechners und er erlaubt eine objektive Kalibrierung.

Zur Kalibrierung des Akkommodationsuntersuchungsgerätes 60 wird mit dem ersten Flüssigkeitslinsensystem 62 eine definierte Brechkraft eingestellt (z.B. -3 dpt) und mittels des Flüssigkeitslinsensystems 64 wird ein definierter Zylinder eingestellt (z.B. 1 dpt 20°).

Anschließend werden die Brechkraft und der Zylinder im Akkomodationsuntersuchungsgerät mit den genannten steuerbaren Linsen kompensiert. Die Kamera 66 nimmt ein Bild auf und der Rechner 68 wertet mittels Bildbearbeitungssoftware dieses Bild aus. Dabei muss die Bildgröße der Soll-Bildgröße entsprechen, ansonsten muss die Kompensation im Akkommodationsuntersuchungsgerät solange fortgesetzt werden bis das mit der Kamera 66 aufgenommene Bild die erwartete Bildgröße erreicht hat. Die Steuerung der Linsen im Akkommodationsmessgerät 60 übernimmt auch der Rechner 68.

Weiterhin kann mit der Anordnung gemäß Figur 4 auch ein Akkommodationsuntersuchungsgerät überprüft werden. Hierzu werden im Akkommodationsuntersuchungsgerät eine definierte Brechkraft (z.B. -3 dpt) und ein definierter Zylinder (z.B. 1 dpt 20°) eingestellt. Das Dioptriefernrohr gemäß Figur 6 wird dann auf die gleiche Brechkraft (z.B. -3 dpt) und den gleichen Zylinder (z.B. 1 dpt 20°) eingestellt. Ist das Akkommodationsuntersuchungsgerät richtig kalibriert, so muss die Bildgröße des mit der Kamera 66 gemessenen Bildes mit dem Soll-Bild übereinstimmen.

Auch das Dioptriefernrohr gemäß Figur 4 kann durch Kombination weiterer Flüssigkeitslinsensysteme, gegebenenfalls mit Glas- oder auch Kunststofflinsen, im Messbereich erweitert und hinsichtlich der Messgenauigkeit durch Vorkompensation verbessert werden.

## Patentansprüche

1. Vorrichtung zum Untersuchen der Akkommodationsfähigkeit eines Auges mit
- einem Flüssigkeitslinsensystem (52, 54) mit einer Mehrzahl von Elektroden (20a, 20b, 20c, 20d, 20e, 20f, 20g, 20h), die um einen Flüssigkeitstropfen (10) herum angeordnet sind,
- einer elektrischen Spannungsversorgung, um wahlweise zur Erzeugung unterschiedlicher elektrischer Felder verschiedene Spannungen an die Elektroden anzulegen,
- einem Bildschirm (56) zur Erzeugung eines Bildes auf der Netzhaut des Auges (50),
- einem Rechner (58) programmiert zum Steuern der an die Elektroden angelegten Spannungen mit Frequenzen im Millisekundenbereich und zum Ändern der Frequenz der erzeugten Bildänderung, wobei das Flüssigkeitslinsensystem zwei Flüssigkeitslinsen (52, 54) aufweist, wobei die eine Flüssigkeltslinse (52) für eine sphärische Korrektur und die andere Flüssigkeitslinse (54) für eine Zylinderkorrektur ansteuerbar sind.

2. Verfahren zum Untersuchen der Akkommodationsfähigkeit eines Auges mit einem Flüssigkeitslinsensystem (52, 54) mit einer Mehrzahl von Elektroden (20a, 20b, 20c, 20d, 20e, 20f, 20g, 20h), die um einen Flüssigkeitstropfen (10) herum angeordnet sind, und mit folgenden Schritten:
- Anlegen von Spannungen an die Elektroden zur Erzeugung von elektrischen Feldern zur Änderung des Flüssigkeitstropfens,
- Verändern der an die Elektroden angelegten Spannungen mit Frequenzen im Millisekundenbereich, und
- untersuchen des auf der Netzhaut abgebildeten Bildes,
wobei das Flüssigkeitslinsensystem zwei Flüssigkeitlinsen (52, 54) aufweist und die eine Flüssigkeitslinse (52) für eine sphärische Korrektur und die andere Flüssigkeitslinse (54) für eine Zyünderkorrektur angesteuert werden.

3. Vorrichtung nach Anspruch 1, wobei der Rechner (58) die Bildgröße und/oder Helligkeit des auf dem Bildschirm (56) dargestellten Bildes abhängig von der Fehlsichtigkeit des Auges steuert.

4. Verfahren nach Anspruch 2, **dadurch gekennzeichnet, dass** die Bildgröße und/oder Helligkeit des Bildes auf dem Bildschirm (58) abhängig von der Fehlsichtigkeit des Auges gesteuert wird.

## Claims

1. Device for examining the accommodation ability of an eye, comprising:
- a liquid-lens system (52, 54) having a plurality of electrodes (20a, 20b, 20c, 20d, 20e, 20f, 20g, 20h) which are arranged around a liquid drop (10),
- an electrical voltage supply for optionally applying various voltages to the electrodes in order to generate different electrical fields,
- a monitor (56) for generating an image on the retina of the eye (50),
- a computer (58) which is programmed to control the voltages applied to the electrodes by means of frequencies in the millisecond range and to change the frequency of the generated image change,
- the liquid-lens system comprising two liquid lenses (52, 54), wherein the one liquid lens (52) may be driven for spherical correction and the other liquid lens (54) may be driven for cylinder correction.

2. Method for examining the accommodation ability of an eye, comprising a liquid-lens system (52, 54) having a plurality of electrodes (20a, 20b, 20c, 20d, 20e, 20f, 20g, 20h) which are arranged around a liquid drop (10), the method having the following steps:
- applying voltages to the electrodes in order to generate electrical fields for changing the liquid drop,
- changing the voltages applied to the electrodes by means of frequencies in the millisecond range, and
- examining the image mapped on the retina,
- the liquid-lens system comprising two liquid lenses (52, 54), wherein the one liquid lens (52) is driven for spherical correction and the other liquid lens (54) is driven for cylinder correction.

3. Device according to claim 1,
wherein the computer (58) controls the size of the image and/or brightness of the image displayed on the monitor (56) as a function of the ametropia of the eye.

4. Method according to claim 2,
**characterized in that** the size of the image and/or brightness of the image displayed on the monitor (58) is controlled as a function of the ametropia of the eye.

## Revendications

1. Appareil pour l'examen de l'accommodation oculaire, comprenant
- un système lenticulaire liquide (52, 54) avec une pluralité d'électrodes (20a, 20b, 20c, 20d, 20e, 20f, 20g, 20h) disposées autour d'une goutte de liquide (10),
- une alimentation en tension électrique pour appliquer au choix différentes tensions aux électrodes afin de créer différents champs électriques,
- un écran (56) pour générer une image sur la rétine de l'oeil (50),
- un calculateur (58) programmé pour commander les tensions appliquées aux électrodes au moyen de fréquences de l'ordre de la milliseconde pour modifier la fréquence de modification de l'image, le système lenticulaire liquide (52, 54) présentant deux lentilles liquides (52, 54), une lentille liquide pouvant être commandée pour une correction sphérique et l'autre lentille liquide (54) pour une correction cylindrique.

2. Procédé pour l'examen de l'accommodation oculaire utilisant un système lenticulaire liquide (52, 54) avec une pluralité d'électrodes (20a, 20b, 20c, 20d, 20e, 20f, 20g, 20h) disposées autour d'une goutte de liquide (10), comprenant les étapes suivantes :
- application de tensions aux électrodes pour créer des champs électriques afin de modifier la goutte de liquide,
- modification des tensions appliquées aux électrodes au moyen de fréquences de l'ordre de la milliseconde, et
- examen de l'image représentée sur la rétine de l'oeil, le système lenticulaire liquide présentant deux lentilles liquides (52, 54), et une lentille liquide (52) étant commandée pour une correction sphérique et l'autre lentille liquide (54) pour une correction cylindrique.

3. Appareil selon la revendication 1, le calculateur (58) commandant la taille de l'image et/ou la luminosité de l'image représentée sur l'écran (56) en fonction de l'amétropie de l'oeil.

4. Procédé selon la revendication 2, **caractérisé en ce que** la taille de l'image et/ou de la luminosité de l'image sur l'écran (58) sont commandées en fonction de l'amétropie de l'oeil.
